**Europäisches Patentamt**

**European Patent Office**

**Office européen des brevets**

(19)

(11) Veröffentlichungsnummer: **0 057 871**
**B1**

(12) **EUROPÄISCHE PATENTSCHRIFT**

(45) Veröffentlichungstag der Patentschrift:
**03.10.84**

(21) Anmeldenummer: **82100608.7**

(22) Anmeldetag: **29.01.82**

(51) Int. Cl.³: **C 07 C 76/02,** C 07 C 79/12,
C 07 C 85/11, C 07 C 87/58,
C 07 C 87/60

(54) Verfahren zur Herstellung von gegebenenfalls p-chlor-substituiertem 2,6-Diaminotoluol.

(30) Priorität: **10.02.81 DE 3104643**

(43) Veröffentlichungstag der Anmeldung:
**18.08.82 Patentblatt 82/33**

(45) Bekanntmachung des Hinweises auf die Patenterteilung:
**03.10.84 Patentblatt 84/40**

(84) Benannte Vertragsstaaten:
**BE CH DE FR GB LI**

(56) Entgegenhaltungen:
**EP - A - 0 002 802**
**DE - A - 2 456 308**
**DE - C - 1 040 012**

**Patent Abstracts of Japan Band 4, Nr. 65, 16. Mai 1980
Seite 113C10
JOURNAL OF THE CHEMICAL SOCIETY Band 79, 1901,
London B. COHEN et al. "The Aluminium-Mercury
Couple. Part III. Chlorination of Aromatic Hydrocarbons
in Presence of the Couple. The Constitution of the
Dichlorotoluenes" Seiten 1111 bis 1134**

(73) Patentinhaber: **BAYER AG, Konzernverwaltung RP
Patentabteilung, D-5090 Leverkusen 1 Bayerwerk (DE)**

(72) Erfinder: **Heise, Klaus-Peter, Dr., Dünnwalder Weg 32,
D-5060 Bergisch-Gladbach 2 (DE)**
Erfinder: **Schneider, Ernst, Dr., Gemarkenstrasse 26,
D-5000 Köln-Dellbrück (DE)**
Erfinder: **Wedemeyer, Karlfried, Dr., Bilharzstrasse 7,
D-5000 Köln-Stammheim (DE)**

## Beschreibung

Die vorliegende Erfindung betrifft ein Verfahren zur Herstellung von gegebenenfalls p-chlorsubstituiertem 2,6-Diaminotoluol aus 3,4-Dichlortoluol.

Aus „J. Chem. Soc.", 79, 1122 (1901) ist bekannt, Dichlortoluol in Gegenwart von konzentrierter Schwefelsäure mit einem 7,7fachen Überschuss an rauchender Salpetersäure bei 100°C zu dinitrieren. Zur Isolierung der Dinitroverbindungen wird das Reaktionsgemisch auf Eis gegeben und nach Filtration fraktioniert aus Alkohol und Eisessig kristallisiert.

Weiterhin ist aus „J. Ind. Chem. Soc.", vol. 33, Nr. 5, S. 331, 334 (1959) bekannt, 3,4-Dichlortoluol mit einem Gemisch aus Schwefelsäure und Salpetersäure zu dinitrieren. Nach diesem Verfahren wird das Reaktionsgemisch einige Stunden auf 120°C erhitzt, dann auf Eis gegossen und das ausgefallene Produkt mit Alkohol oder Essigsäure umkristallisiert. Man erhält das 3,4-Dichlor-2,6-dinitrotoluol zu 80%.

Die geschilderten Nitrierverfahren haben den Nachteil, dass drastische Nitrierbedingungen angewandt werden, die sich negativ auf die Ausbeute an gewünschter Dinitroverbindung auswirken. Zur Isolierung der gewünschten Dinitroverbindung ist es erforderlich, z.B. eine technisch aufwendige fraktionierte Kristallisation mit Alkohol/Eisessig durchzuführen. Ausserdem können sich aufgrund der drastischen Nitrierbedingungen sicherheitstechnische Probleme ergeben. Weiterhin erfordert die Beseitigung der noch grosse Mengen nichtumgesetzter Salpetersäure enthaltende Abfallsäure einen erheblichen technischen Aufwand, was die Wirtschaftlichkeit der Nitrierverfahren stark beeinträchtigt.

Es wurde nun ein Verfahren zur Herstellung von gegebenenfalls p-chlorsubstituiertem 2,6-Diaminotoluol gefunden, das dadurch gekennzeichnet ist, dass man 3,4-Dichlortoluol in Gegenwart eines inerten, mit Wasser nicht mischbaren, organischen Lösungs- und/oder Verdünnungsmittels bei Temperaturen von −10 bis + 100°C dinitriert und anschliessend, gegebenenfalls nach Zwischenisolierung der Dinitroverbindung, unter teilweiser oder vollständiger Abspaltung der Chloratome in Gegenwart von inerten organischen Lösungs- und/oder Verdünnungsmitteln und/oder Wasser zur Diaminoverbindung reduziert.

Die Nitrierung des 3,4-Dichlortoluols wird nach dem erfindungsgemässen Verfahren in Gegenwart von inerten, mit Wasser nicht mischbaren, organischen Lösungs- und/oder Verdünnungsmitteln durchgeführt. Dabei können die Lösungs- und/oder Verdünnungsmittel sowohl einzeln als auch im Gemisch untereinander eingesetzt werden.

Als inerte, mit Wasser nicht mischbare, organische Lösungs- und/oder Verdünnungsmittel kommen z.B. aliphatische oder cycloaliphatische Kohlenwasserstoffe mit 5 bis 20, bevorzugt 6 bis 15 Kohlenstoffatomen, wie Hexan, Heptan, Octan, Decan, Dodecan, Decalin, Cyclohexan sowie deren Isomere, bevorzugt Dodecanisomerengemische sowie halogenierte aliphatische Kohlenwasserstoffe mit 1 bis 3 Kohlenstoffatomen, wie Dichlormethan, Trichlormethan, Tetrachlormethan, Dichlorethan, Trichlorethan, Dichlorpropan oder Trichlorpropan, bevorzugt Dichlormethan oder Dichlorethan, in Frage.

Die Menge der inerten organischen Lösungs- und/oder Verdünnungsmittel kann in weiten Bereichen variiert werden. Zum Beispiel werden bei Verwendung von Dichlormethan 0,8 bis 2,5, bevorzugt 1 bis 2 l, bei Verwendung von Dodecan 3 bis 10, bevorzugt 4 bis 7,5 l, bezogen auf 1 kg 3,4-Dichlortoluol, in das erfindungsgemässe Verfahren eingesetzt.

Als Nitriermittel finden bei dem erfindungsgemässen Verfahren vor allem Gemische aus Schwefelsäure und Salpetersäure sowie Gemische aus Oleum und Salpetersäure Verwendung. Weiterhin ist es möglich, die Nitrierung des 3,4-Dichlortoluols mit Nitroniumsalzen, die in Substanz z.B. als Nitroniumtetrafluorborat eingesetzt oder aus Stickoxiden und geeigneten Säuren, wie Distickstoffpentoxid und Schwefelsäure, erzeugt werden können, durchzuführen.

Die Schwefelsäure und Salpetersäure werden vorzugsweise in möglichst wasserfreier Form eingesetzt (z.B. in Form von 98%iger Schwefel- und Salpetersäure oder Monohydrat).

Als Oleum werden bevorzugt die üblichen, leicht handhabbaren Gemische aus konzentrierter Schwefelsäure und $SO_3$, die einen Gehalt an $SO_3$ von 20 oder 65 Gew.-% haben, eingesetzt.

Nach dem erfindungsgemässen Verfahren ist es nicht erforderlich, die Nitrierung mit einem grossen Überschuss an Nitrierreagenz durchzuführen. Es genügt bereits, die Salpetersäure bzw. die Nitroniumsalze in der für die Dinitrierung theoretisch benötigten Menge von 2 mol, bezogen auf das Dichlortoluol, einzusetzen. Selbstverständlich ist es auch möglich, eine grössere Menge an Salpetersäure bzw. Nitroniumsalzen einzusetzen. In der Praxis arbeitet man mit etwa 2 bis 4, bevorzugt 2,05 bis 3,5, mol, Salpetersäure bzw. Nitroniumsalzen, bezogen auf 1 mol Dichlortoluol.

Die zur Dinitrierung verwendete Menge an Schwefelsäure bzw. Oleum kann in weiten Bereichen variiert werden und ist durch einen Vorversuch leicht zu ermitteln. Sie richtet sich vor allem nach dem Wassergehalt der eingesetzten Schwefelsäure und/oder Salpetersäure sowie dem $SO_3$-Gehalt des verwendeten Oleums.

Die sich an die Dinitrierung des 3,4-Dichlortoluols anschliessende Reduktion mit teilweiser oder vollständiger Abspaltung der Chloratome kann nach dem erfindungsgemässen Verfahren in Gegenwart eines unter den Reaktionsbedingungen inerten organischen Lösungs- und/oder Verdünnungsmittels und/oder in Gegenwart von Wasser mit gegebenenfalls auf Trägern aufgebrachten Raney-Katalysatoren oder Edelmetallen in elementarer oder gebundener Form, gegebenenfalls in Anwesenheit eines geeigneten Salzsäureakzeptors, oder mit unedlen Metallen im sauren Medium durchgeführt werden.

Als inerte organische Lösungs- und/oder Verdünnungsmittel können bei der Reduktion aliphatische, cycloaliphatische, aromatische oder araliphatische Kohlenwasserstoffe, mit bis zu 20, bevorzugt 6 bis 15 C-Atomen, wie Hexan, Heptan, Octan, Dodecan, Cyclohexan, Decalin, Benzol, Toluol, Xylol sowie deren Isomere; aliphatische, cycloaliphatische oder aromatische Ether, mit bis zu 14, bevorzugt mit 4 bis 8 C-Atomen, wie Methylbutylether, Tetrahydrofuran, Aniso, 1,4-Dioxan sowie deren Isomere; aliphatische oder cycloaliphatische Alkohole oder Phenole, mit bis zu 12, bevorzugt 1 bis 8 C-Atome, wie Methanol, Ethanol, Propanol, Butanol, Pentanol, Cyclohexanol, Phenol und deren Isomere, sowie aliphatische Carbonsäuren, Carbonsäureester oder Carbonsäureamide, mit bis zu 12, bevorzugt mit 2 bis 6 C-Atomen, wie Essigsäure, Ethylacetat, Butylacetat, Dimethylformamid und Dimethylacetamid eingesetzt werden.

Weiterhin ist es möglich, bei der Reduktion in Gegenwart von Wasser zu arbeiten.

Bevorzugt werden bei der Reduktion niedere Alkohole, wie Methanol, Ethanol, Propanol und/oder Butanol sowie deren Isomere und/oder Wasser eingesetzt.

Dabei können die Lösungs- und/oder Verdünnungsmittel sowohl einzeln als auch im Gemisch untereinander eingesetzt werden.

Die Menge der inerten organischen Lösungs- und/oder Verdünnungsmittel bzw. des Wassers ist nicht kritisch und kann in weiten Bereichen schwanken. Die optimale Menge kann leicht durch Vorversuche ermittelt werden.

Als Raney-Katalysatoren können gebräuchliche Raney-Kontakte, wie Raney-Nickel, Raney-Nickel/Eisen, Raney-Nickel/Kobalt, Raney-Nickel/Kupfer oder Raney-Kobalt, in das erfindungsgemässe Verfahren eingesetzt werden. Bevorzugt werden Raney-Nickelkatalysatoren verwendet. Als Edelmetalle seien die Elemente der 8. Gruppe des Periodensystems der Elemente (Mendelejew), wie Ruthenium, Rhodium, Palladium und Platin, bevorzugt Palladium und Platin, genannt.

Als Edelmetalle in gebundener Form können beispielsweise die Oxide oder Halogenide eingesetzt werden.

Die Edelmetallkatalysatoren können auf Trägermaterialien aufgebracht sein. Dafür kommen alle an sich bekannten Trägermaterialien in Frage, soweit sie sich unter den Reaktionsbedingungen inert verhalten. Als solche seien Bariumsulfat, Aluminiumoxide, Silikate und Kohlenstoff in verschiedenen Formen, bevorzugt Aktivkohle, genannt.

Besonders bei der kontinuierlichen Durchführung des erfindungsgemässen Verfahrens ist es vorteilhaft, den Edelmetallkatalysator auf einem Trägermaterial im Reaktionsraum als Festbett- oder Fliessbettkatalysator anzuordnen.

Zur vollständigen Abspaltung der Chloratome setzt man als Katalysator bevorzugt Raney-Nickel oder Palladium, zur partiellen Enthalogenierung als Katalysator bevorzugt Platin, ein.

Die Katalysatoren, die zur Durchführung des erfindungsgemässen Verfahrens eingesetzt werden, behalten auch bei wiederholter Verwendung oder bei kontinuierlicher Durchführung des erfindungsgemässen Verfahrens über lange Zeit ihre Aktivität und ihre Selektivitäten und ergeben gleichbleibend hohe Ausbeuten.

Die Menge der einzusetzenden Katalysatoren beträgt im allgemeinen 0,01 bis 30, bevorzugt 0,3 bis 20 Gew.-%, bezogen auf das als Ausgangsmaterial eingesetzte 3,4-Dichlortoluol. Bei Verwendung eines auf einen Träger aufgebrachten Katalysators richtet sich die einzusetzende Kontaktmenge nach dem Gewichtsverhältnis von Trägermaterial zu Katalysator. Sie wird dabei so bemessen, dass die Menge des Katalysators den oben angegebenen Mengen des einzusetzenden Katalysators entspricht.

Erfolgt die Reduktion und die Enthalogenierung des Dinitrodichlortoluols katalytisch mit Wasserstoff in Gegenwart der oben beschriebenen Raney-Katalysatoren oder der Edelmetallkatalysatoren, so arbeitet man im allgemeinen bei Wasserstoffdrücken von 1 bis 300, bevorzugt bei 2 bis 200 bar.

Die Temperaturen können dabei in weiten Grenzen schwanken und hängen, wie der Wasserstoffdruck, vor allem von dem für die Nitrierung verwendeten Lösungs- und/oder Verdünnungsmittel sowie von dem jeweils eingesetzten Salzsäureakzeptor ab. Üblicherweise hydriert man bei 20 bis 250, bevorzugt bei 30 bis 200° C.

Zur vollständigen Abspaltung der Chloratome bei der Reduktion des dinitrierten 3,4-Dichlortoluols werden Verbindungen zugesetzt, die die bei der Reduktion freiwerdende Salzsäure binden oder puffern. Für diesen Zweck sind z.B. Basen, wie Metalloxide, -hydroxide, -carbonate sowie Salze organischer Säuren geeignet. So können Alkalioxide, Alkalihydroxide, Alkalicarbonate, Erdalkalioxide, Erdalkalihydroxide oder Erdalkalicarbonate, wie Calciumoxid, Calciumhydroxid, Calciumcarbonat, Bariumoxid, Bariumhydroxid, Natriumhydroxid, Natriumcarbonat, Kaliumhydroxid, Kaliumcarbonat sowie Ammoniak und Ammoniumhydroxid, weiterhin Acetate, wie Ammonium- oder Natriumacetat, als Säureakzeptoren sowohl einzeln als auch im Gemisch untereinander eingesetzt werden. Die Salzsäureakzeptoren können dem Reaktionsgemisch sowohl in Substanz als auch in Lösung bzw. Suspension in einem für die Reduktion geeigneten inerten Lösungs- und/oder Verdünnungsmittel zugesetzt werden.

Man kann die salzsäurebindenden Mittel in geringem Unterschuss oder im Überschuss, bezogen auf die bei der Entchlorierung entstehende Menge an Salzsäure, einsetzen. Bevorzugt werden die säurebindenden Mittel jedoch in etwa äquivalenter Menge zugegeben.

Das erfindungsgemässe Verfahren kann in verschiedener Weise duchgeführt werden. So ist es möglich, die Dinitrierung des 3,4-Dichlortoluols und die Reduktion der entsprechenden Dinitroverbindung ohne Zwischenisolierung der dinitrierten Verbindung in einem sogenannten Eintopfverfahren durchzuführen oder aber die Nitroverbindung zunächst zu isolieren und anschliessend dieselbe in einer oder in mehreren Stufen zum 2,6-Diaminotoluol bzw. 2,6-Diamino-4-chlortoluol zu reduzieren.

Bei der Verfahrensweise mit der Zwischenisolierung des 3,4-Dichlor-2,6-dinitrotoluols kann man beispielsweise so vorgehen, dass man bei Temperaturen von etwa 30 bis 40°C das 3,4-Dichlortoluol in einem Dodekanisomerengemisch mit einem Schwefelsäure/Salpetersäuregemisch nitriert und das Dichlordinitrotoluol z.B. durch Filtrieren des Reaktionsgemisches isoliert. Nach Phasentrennung kann sowohl das organische Lösungsmittel vollständig als auch die bei der Nitrierung anfallende Abfallsäure (Gemisch aus Schwefelsäure, Salpetersäure und Wasser), gegebenenfalls nach Zusatz von wasserbindenden Mitteln, wie Oleum, zumindest teilweise rückgeführt werden. Durch diese Kreisführung der Lösungs- und/oder Verdünnungsmittel und der Abfallsäure können erhebliche Mengen an Lösungs- und/oder Verdünnungsmittel sowie an Schwefelsäure eingespart werden. Weiterhin gestattet die Rückführung des mit Produkt gesättigten Lösungs- und/oder Verdünnungsmittels, das Nitrierprodukt praktisch quantitativ, bezogen auf das eingesetzte Dichlortoluol, in fester Form zu isolieren.

Bei Ansprüchen höchster Reinheit lässt sich das 3,4-Dichlor-2,6-dinitrotoluol durch Kristallisation, Umlösen oder bevorzugt durch Auskochen mit einem geeigneten Lösungsmittel, z.B. Methanol, mit einer Reinheit von über 99% gewinnen.

Die Nitrierung des 3,4-Dichlortoluols kann in verschiedener Weise durchgeführt werden. So ist es z.B. möglich, das Nitriergut zusammen mit dem inerten organischen Lösungs- und/oder Verdünnungsmittel vorzulegen und das Nitriermittel zuzugeben oder aber das Nitriermittel vorzulegen und das zu nitrierende Produkt entweder in Substanz oder gelöst oder suspendiert in einem inerten organischen Lösungs- und/oder Verdünnungsmittel zuzudosieren. Weiterhin können das Nitriermittel und das in einem Lösungs- und/oder Verdünnungsmittel gelöste oder suspendierte Nitriergut gleichzeitig zu bereits teilweise oder vollständig nitriertem Produkt zugegeben werden. Bevorzugt wird die Nitrierung so durchgeführt, dass man das zu nitrierende Produkt mit dem inerten organischen Lösungs- und/oder Verdünnungsmittel vorlegt und das Nitriermittel zugibt.

Nach dem erfindungsgemässen Verfahren kann das 3,4-Dichlor-2,6-dinitrotoluol, wie zuvor erwähnt, in einer oder mehreren Stufen zum 2,6-Diaminotoluol oder zum 2,6-Diamino-4-chlortoluol reduziert werden.

Bei der einstufigen Verfahrensweise reduziert man das 3,4-Dichlor-2,6-dinitrotoluol bevorzugt katalytisch mit Raney-Katalysatoren oder mit Edelmetallkatalysatoren in Gegenwart von unter den Reaktionsbedingungen inerten organischen Lösungs- und/oder Verdünnungsmitteln und gegebenenfalls in Gegenwart von geeigneten Salzsäureakzeptoren.

So erhält man beispielsweise das 2,6-Diaminotoluol in guten Ausbeuten, wenn man 3,4-Dichlor-2,6-dinitrotoluol mit Raney-Nickel in Isopropanol in Gegenwart von Calciumoxid bei Temperaturen von 50 bis 150°C und bei einem Wasserstoffdruck von 10 bis 30 bar hydriert.

Die einstufige Reduktion zum 2,6-Diamino-4-chlortoluol gelingt ebenfalls in guten Ausbeuten und Reinheiten, wenn man z.B. 3,4-Dichlor-2,6-dinitrotoluol mit Platin in Gegenwart von niederen Alkoholen bei Temperaturen von etwa 100 bis 130°C und einem Wasserstoffdruck von etwa 5 bis 15 bar reduziert. Der Zusatz eines Salzsäureakzeptors ist unter diesen Reaktionsbedingungen nicht unbedingt erforderlich.

Bei der mehrstufigen Verfahrensweise wird das 3,4-Dichlor-2,6-dinitrotoluol zunächst zur entsprechenden Diaminoverbindung reduziert, wobei diese gegebenenfalls zwischenisoliert werden kann, und anschliessend teilweise oder vollständig enthalogeniert. Die vollständige Enthalogenierung der Diaminotoluole wird nach dem erfindungsgemässen Verfahren unter Zugabe von Verbindungen durchgeführt, die die bei der Chlorabspaltung freiwerdende Salzsäure zu binden oder zu puffern vermögen.

Dabei kann die Reduktion der Nitrogruppen katalytisch mit Raney-Katalysatoren, z.B. Raney-Nickel, in Gegenwart von Wasserstoff oder Hydrazin oder mit Edelmetallkatalysatoren, wie Platin oder Palladium, in Gegenwart von Wasserstoff und eines unter den Reaktionsbedingungen inerten Lösungs- und/oder Verdünnungsmittels durchgeführt werden. Daneben sind auch andere übliche Methoden zur Reduktion von Nitrogruppen möglich, wie die Reduktion mit unedlen Metallen, z.B. mit Eisen, Zink oder Zinn.

Will man beispielsweise das Diaminodichlortoluol vor Abspaltung der Chloratome als Zwischenprodukt isolieren, so reduziert man die Nitrogruppen des Dinitrodichlortoluols vorzugsweise mit Raney-Nickel, Platin oder Palladium, besonders bevorzugt mit Platin, in Gegenwart von Alkoholen. Dabei werden Temperatur und Wasserstoffdruck so gewählt, dass die Chlorabspaltung möglichst unterdrückt wird. Um die Enthalogenierung weitestgehend zurückzudrängen, kann es weiterhin vorteilhaft sein, dem Reaktionsgemisch in katalytischen Mengen Katalysatorregler, das sind beispielsweise basisch reagierende Substanzen, wie Morpholin, Calciumcarbonat und/oder Calciumoxid sowie Bleiverbindungen, wie Bleiacetat, oder Schwefelverbindungen, wie Bis-(2-hydroxyethyl)sulfid, zuzusetzen.

Durch Vorversuche kann die optimale Menge der zuzusetzenden Katalysatorregler sowie die optimale Temperatur und der optimale Wasserstoffdruck leicht ermittelt werden.

Bei der Reduktion des Dichlordinitrotoluols ohne Zwischenisolierung des entsprechenden Dichlordiaminotoluols geht man z.B. so vor, dass man die Dinitroverbindung mit geringem Wasserstoffüberdruck (ca. 1 bis 10 bar) und bei Temperaturen von ca. 20 bis 60°C in Gegenwart von Raney-Nickel oder Palladium bevorzugt in Alkoholen oder Alkohol/Wasser-Gemischen hydriert. Unter diesen Hydrierbedingungen findet eine Chlorabspaltung nur in geringem Masse statt, so dass Korrosionsprobleme nicht zu befürchten sind. Um aber die Chlorabspaltung noch weiter unterdrükken zu können, können, wie zuvor erwähnt, dem Reaktionsgemisch noch Katalysatorregler zugesetzt werden.

Zur vollständigen Hydrogenolyse der Chloratome setzt man nach dem erfindungsgemässen Verfahren der Diaminodichlortoluollösung oder -suspension Verbindungen zu, die die bei der Hydrierung freiwerdende Salzsäure binden oder puffern. Diese können dem Reaktionsgemisch in Substanz oder als Lösung bzw. Suspension in einem für die Hydrierung geeigneten, inerten Lösungs- und/ oder Verdünnungsmittel zugesetzt werden. Dabei bestimmen das eingesetzte Lösungs- und/oder Verdünnungsmittel und insbesondere die eingesetzte Base die zur vollständigen Chlorabspaltung optimalen Temperatur- und Druckparameter. Das bedeutet, dass sich ein weites Spektrum von Temperatur- und Druckgrössen findet, unter denen 2,6-Diaminotoluol in guten Ausbeuten und Qualitäten gewonnen werden kann.

Steht beispielsweise eine Hydrieranlage zur Verfügung, die nur für den Niederdruckbereich geeignet ist, so empfiehlt sich, insbesondere Alkali- und/oder Erdalkalihydroxide- und/oder -oxide als Säureakzeptoren zu verwenden. So kann man z.B. die vollständige Enthalogenierung von Diaminodichlortoluol in alkoholischer oder wässerig-alkoholischer Lösung oder -suspension nach Zusatz von äquimolaren Mengen Natronlauge bereits bei geringem Wasserstoffdruck (ca. 2 bis 10 bar) und bei Temperaturen unter 50°C, durchführen. Bei Anwendung höherer Temperaturen beschleunigt sich die Chlorabspaltung. Z.B. verläuft sie bei 80° anstelle von 50°C mehr als doppelt so schnell.

Ist andererseits eine Hydrierapparatur vorhanden, die die Durchführung der Reaktion bei hohen Wasserstoffdrücken ermöglicht, so kann die Dehalogenierung auch bei Drücken über 15 bar (z.B. 15 bis 200 bar) und bei höheren Temperaturen (z.B. 50 bis 200°C) erfolgen.

Will man eine nur teilweise Enthalogenierung des 3,4-Dichlor-2,6-dinitrotoluols zum 2,6-Diamino-4-chlortoluol durchführen, so kann die partielle Chlorabspaltung durch Zugabe von Verbindungen, wie Morpholin, Ammoniumchlorid oder Ammoniumacetat, gesteuert und dadurch die Selektivität des Enthalogenierungsmittels deutlich verbessert werden.

Dabei hängen die Hydrierbedingungen von der Art der zugesetzten Verbindungen ab. Je nach Art der Verbindung sind milde oder drastische Hydrierbedingungen zu wählen, damit partielle Chlorabspaltung erfolgt. Setzt man dem Reaktionsgemisch beispielsweise Morpholin zu, so kann bei einem Wasserstoffdruck von etwa 3 bis 7 bar und bei Temperaturen von etwa 80 bis 100°C hydriert werden. Bei Zugabe von Ammoniumchlorid kann man bei 10 bis 30 bar und bei Temperaturen von 70 bis 110°C die partielle Enthalogenierung durchführen, während z.B. bei Zusatz von Ammoniumacetat ein Wasserstoffdruck von etwa 150 bis 200 bar und Temperaturen von etwa 130 bis 170°C vorteilhaft sind.

Die partielle Dechlorierung des 3,4-Dichlor-2,6-dinitrotoluols kann anstatt mit Raney-Katalysatoren oder Edelmetallkatalysatoren auch mit unedlen Metallen in saurem Medium durchgeführt werden. Dabei wird z.B. die Dichlordinitroverbindung in wässeriger Salzsäure mit Eisen, Zink oder Zinn reduziert und teilweise enthalogeniert.

Die Reduktion des 3,4-Dichlor-2,6-dinitrotoluols zum 2,6-Diaminotoluol bzw. 2,6-Diamino-4-chlortoluol kann entweder nur in einem Autoklaven erfolgen, wobei der Katalysator zweckmässigerweise nach durchgeführter Entchlorierung alkalifrei gewaschen und in den Hydrierprozess zurückgeführt wird, oder aber in zwei getrennten Reaktoren, wobei jeweils unter Katalysatorrückführung in einem Reaktor nur die Nitrogruppen reduziert werden, während in dem anderen ausschliesslich die Enthalogenierung durchgeführt wird. In letzterem Fall entfällt das alkalifreie Waschen des Katalysators vor dessen Rückführung.

Bei der in einem sogenannten Eintopfverfahren durchzuführenden Nitrierung, anschliessender Reduktion und teilweiser oder vollständiger Enthalogenierung des 3,4-Dichlortoluols geht man im allgemeinen so vor, dass man die Nitrierung in Gegenwart eines inerten organischen Lösungs- und/oder Verdünnungsmittels, beispielsweise einem Dodekanisomerengemisch, durchführt, nach Abtrennen der Abfallsäure das im organischen Lösungsmittel gelöste Nitrierprodukt mit Katalysator und gegebenenfalls mit einem der zuvor aufgeführten Zuschlagsstoffe versetzt und die Reduktion und Enthalogenierung, wie zuvor beschrieben, durchführt.

Die Aufarbeitung des in verschiedener Weise erhaltenen Reaktionsgemisches kann in üblicher Weise erfolgen.

Wurde in Gegenwart von salzsäurebindenden Zuschlagsstoffen gearbeitet, so kann man beispielsweise durch Filtrieren zunächst den Katalysator und gegebenenfalls ausgefallene Salze aus dem Reaktionsgemisch entfernen und dann nach dem Abtrennen des inerten organischen Lösungs- und/oder Verdünnungsmittels das 2,6-Diaminotoluol bzw. das 2,6-Diamino-4-chlortoluol in bekannter Weise vom verbliebenen Salz separieren. Hierzu kann man z.B. dem Reaktionsgemisch ein Lösungsmittel zusetzen, in dem das Diaminotoluol löslich, nicht aber das Salz löslich ist oder aber das Salz in Wasser lösen und das 2,6-Diaminotoluol mit einem geeigneten Lösungsmittel extrahieren oder aus der wässerigen Phase das Diaminotoluol kristallisieren.

Das erfindungsgemässe Verfahren kann sowohl kontinuierlich als auch diskontinuierlich durchgeführt werden.

Nach dem erfindungsgemässen Verfahren ist das gegebenenfalls p-chlorsubstituierte 2,6-Diaminotoluol in einfacher Weise in hohen Ausbeuten und guten Reinheiten zugänglich. Nebenreaktionen wurden praktisch nicht beobachtet. Dadurch entfallen technisch aufwendige und

kostspielige Kristallisations- und Reinigungs-operationen. Aufgrund der milden Nitrierbedin-gungen und aufgrund der möglichen Rückführung und Wiederverwendung des nach der Nitrie-rung anfallenden Lösungsmittels und gegebenen-falls der Abfallsäure sowie der eingesetzten Katalysatoren stellt sich das erfindungsgemässe Ver-fahren als umweltfreundlich und sicher-heitstechnisch leicht zu handhabendes Verfahren dar.

2,6-Diaminotoluol und 2,6-Diamino-4-chlortoluol sind z.B. wertvolle Zwischenprodukte für die Herstellung von Diisocyanaten bzw. Polyurethanen (US-PS Nr. 3203931); das 2,6-Diaminotoluol wird ausserdem für die Herstellung von Farbstoffen und Antioxidantien verwendet (US-PS Nr. 2765348).

Die nachfolgenden Beispiele sollen das erfindungsgemässe Verfahren verdeutlichen, ohne es jedoch auf diese Beispiele einzuschränken.

### Beispiel 1

Zu 644 g (4 mol) 3,4-Dichlortoluol in 1200 ml Dichlormethan wird bei 0°C Nitriersäure aus 643 g (10,2 mol) 98%iger Salpetersäure und 3064 g 98%iger Schwefelsäure zugetropft. Anschliessend erhöht man die Temperatur auf 40°C und rührt nach, bis völlige Umsetzung zum Dichlordinitrotoluol erfolgt ist. Nach Phasentrennung wird die Absäure mit Dichlormethan extrahiert, die organischen Phasen vereinigt und mit Sodalösung neutral gewaschen. Nach Abtreiben des Lösungsmittels und Trocken verbleiben 997 g kristalliner Rückstand vom Schmelzpunkt = Fp. 90 bis 91°C. Ausbeute, bezogen auf 3,4-Dichlortoluol: 99,3% der Theorie; Reinheit (GC): 95,9% 3,4-Dichlor-2,6-dinitrotoluol; 3,9% 4,5-Dichlor-2,3-dinitroto-luol.

Zur Abtrennung der geringen Mengen an Dinitroisomeren kann das Rohprodukt in 2290 ml Methanol suspendiert und 30 min bei Rückflusstemperatur gehalten werden. Nach Abkühlen auf Raumtemperatur wird der Kristallbrei abgesaugt, mit 498 ml eiskaltem Methanol nachgewaschen und getrocknet. Ausbeute 924 g, Schmelzpunkt = Fp. 92 bis 93°C, Reinheit (GC) 99,5%.

### Beispiel 2

Zu ehalten werden. Nach Abkühlen auf Raumtemperatur wird der Kristallbrei abgesaugt, mit 498 ml eiskaltem Methanol nachgewaschen und getrocknet. Ausbeute 924 g, Schmelzpunkt = Fp. 92 bis 93°C, Reinheit (GC) 99,5%.

### Beispiel 2

Zu 200 g (1,24 mol) 3,4-Dichlortoluol in 1400 ml technischem Isododecan wird bei 33 bis 36°C Mischsäure aus 164 g (2,55 mol) 98%iger Salpetersäure und 860 g Monohydrat zugetropft. Man rührt bei gleicher Temperatur so lange nach, bis völlige Umsetzung eingetreten ist. Man lässt das Reaktionsgemisch in 2400 ml kaltes Wasser laufen. Nach Abfiltrieren wird der Rückstand mit Soda neutral gestellt und mit Hilfe von Wasserdampf vom Lösungsmittel befreit. Zusammen mit dem in der Isododecanmutterlauge befindlichen Anteil beträgt die Ausbeute an Dichlordinitrotoluol 301 g. Reinheit (GC): 94,4% 3,4-Dichlor-2,6-dinitrotoluol; 4,5% 4,5-Dichlor-2,3-dinitroto-luol.

Das Nitrierprodukt, Gehalt an 4,5-Dichlor-2,3-dinitrotoluol 4,5%, wird in 2150 ml Methanol suspendiert und in einem mit Rührer versehenen Autoklaven mit 8,5 g CaCO$_3$ und 60 g Ra-Nickel (wasserfeucht) versetzt. Nach Spülen des Autoklaven mit Argon erfolgt die Reduktion der Nitrogruppen in Gegenwart von Wasserstoff bei einem Gesamtdruck von 4 bar und einer Temperatur von 50°C. Dabei wird der Druck durch Aufpressen von Wasserstoff konstant gehalten. Die Chlorabspaltung erfolgt nach Einpumpen von 96 g (2,4 mol) NaOH in 700 ml Wasser bei gleichem Druck und einer Temperatur von 80°C. Zur Aufarbeitung wird vom Kontakt abfiltriert und der Alkohol bei Normaldruck abdestilliert. Das Produkt wird nach Erkalten abfiltriert; restliche Mengen an Produkt durch Extraktion mit Dichlormethan isoliert. Nach Übertreiben bei 45 mbar/185°C erhält man 136,5 g (90,2% der Theorie, bezogen auf 3,4-Dichlortoluol) farbloses 2,6-Diaminotoluol, dessen Reinheit (GC) 97,0% beträgt.

Bei Einsatz von 99,5%igem 3,4-Dichlor-2,6-dinitrotoluol, hergestellt nach Beispiel 1, beträgt die Reinheit des analog vorstehender Beschreibung erhaltenen 2,6-Diaminotoluols 99,7% bei gleicher Ausbeute.

### Beispiel 3

161 g (1 mol) 3,4-Dichlortoluol, Gehalt 99,5%, werden mit 1160 ml technischem Isododecan verdünnt und bei Raumtemperatur beginnend mit aus 206 g (3,2 mol) 98%iger Salpetersäure und 770 g 98%iger Schwefelsäure hergestellter Mischsäure so versetzt, dass die Temperatur 33 bis 36°C nicht übersteigt. Es wird 1 h nachgerührt, dann scharf abgesaugt. Das durch Filtration isolierte Rohprodukt wird nach Waschen mit Wasser durch Ausblasen mit Wasserdampf vom anhaftenden Isododecan befreit, wobei zum Entfernen restlicher Mengen Säure Soda zugesetzt wird.

Die Gesamtausbeute beträgt 99,3% der Theorie, bezogen auf 3,4-Dichlortoluol, die Selektivität für 3,4-Dichlor-2,6-dinitrotoluol 95,6%. Bei zehnma-liger Rückführung der Isododecanmutterlauge be-trägt die durch Filtration isolierte Menge an Dichlordinitrotoluol im Schnitt 98,5% der Theorie.

### Beispiel 4

161 g (1 mol) 3,4-Dichlortoluol in 1000 ml Isododecan werden mit einer aus 193 g (3 mol) 98%iger Salpetersäure und 246 g 65%igem Oleum bereiteten Mischsäure bei 30 bis 33°C nitriert. Nach Aufarbeitung durch Absaugen des Reak-tionsgemisches erhält man das Dichlordinitro-toluol in einer Ausbeute von 94,4% der Theorie, bezogen auf 3,4-Dichlortoluol.

### Beispiel 5

Zu 161 g (1 mol) 3,4-Dichlortoluol in 1000 ml technischem Isododecan wird bei 33 bis 35°C Mischsäure aus 144 g (2,25 mol) Salpetersäure (98%ig) und 490 g Monohydrat zugetropft. Bei gleicher Temperatur wird 2 h lang nachgerührt. Anschliessend filtriert man das ausgefallene Nitrierprodukt direkt ab und trennt aus dem Filtrat die Abfallsäure ab. Filterkuchen und Mutterlauge werden wie im Beispiel 2 beschrieben, aufgearbeitet. Zusammen mit dem Anteil aus der Isododecanphase wird eine Ausbeute an Dichlordinitrotoluol von 98,5% erzielt. Die Selektivität beträgt 92,2%.

### Beispiel 6

50,2 g (0,2 mol) 3,4-Dichlor-2,6-dinitrotoluol werden in 200 ml Isopropanol in Gegenwart von 11,2 g CaO und 5 g Ra-Nickel (wasserfeucht) in einem Autoklaven unter Rühren bei 50°C und mit Wasserstoff bei 10 bar Gesamtdruck hydriert. Nach Verlangsamung der Wasserstoffaufnahme wird die Temperatur bis auf 150°C, der Druck auf 30 bar erhöht. Der Gehalt des Hydrierproduktes an 2,6-Diaminotoluol beträgt 87,2%.

### Beispiel 7

25,1 g (0,1 mol) 3,4-Dichlor-2,6-dinitrotoluol werden zusammen mit 200 ml Methanol, 5 g Ra-Nickel (wasserfeucht) und 0,7 g $CaCO_3$ mit Wasserstoff bei 15 bar Gesamtdruck und einer Temperatur von 50°C hydriert, bis kein Dichlordinitrotoluol mehr nachweisbar ist. Nach Zusatz von 8,4 g (0,21 mol) NaOH in 100 ml Methanol wird unter den gleichen Bedingungen enthalogeniert. Die Ausbeute an 2,6-Diaminotoluol beträgt 95,6%.

### Beispiel 8

Es wird analog Beispiel 7 gearbeitet, jedoch unter Verwendung von 3 g Palladium (1%ig) auf Eponit®-Kohle als Katalysator. Ausbeute an 2,6-Diaminotoluol: 94,7%.

### Beispiel 9

15,6 g (0,1 mol) 2,6-Diamino-4-chlortoluol, hergestellt nach Beispiel 11, werden in 250 ml Methanol mit 5 g Ra-Nickel (wasserfeucht) und 9 g (0,11 mol) Natriumacetat versetzt und bei 100°C mit Wasserstoff bei einem Gesamtdruck von 50 bar enthalogeniert. Gehalt des Hydrierprodukts an 2,6-Diaminotoluol: 88,1%.

### Beispiel 10

19,1 g (0,1 mol) 2,6-Diamino-3,4-dichlortoluol werden in 500 ml Isopropanol mit 3,1 g Palladium auf Kohle (1%ig) und 6,2 g (0,11 mol) CaO bei 120°C mit Wasserstoff bei einem Gesamtdruck von 50 bar enthalogeniert. Ausbeute an 2,6-Diaminotoluol: 91,2%.

### Beispiel 11

Im Autoklaven werden 50 ml Methanol, 12,3 g (0,22 mol) CaO und 2 g Platin auf Eponit®-Kohle (1%ig) vorgelegt und dann eine Lösung von 19,1 g (0,1 mol) 2,6-Diamino-3,4-dichlortoluol in 200 ml Methanol mit einer Geschwindigkeit von 2 ml/min zugepumpt und hydriert. Der Gesamtdruck im Autoklaven beträgt dabei 120 bar, die Temperatur 180°C.

Nach Abtrennen vom Kontakt wird das Reaktionsgemisch vollständig vom Lösungsmittel und vom Reaktionswasser befreit, der verbliebene Rückstand m hydriert. Der Gesamtdruck im Autoklaven beträgt dabei 120 bar, die Temperatur 180°C.

Nach Abtrennen vom Kontakt wird das Reaktionsgemisch vollständig vom Lösungsmittel und vom Reaktionswasser befreit, der verbliebene Rückstand mit trockenem Methanol aufgenommen, vom ungelösten Salz abfiltriert und das Methanol im Vakuum entfernt.

Man erhält 2,6-Diamino-4-chlortoluol in einer Ausbeute von 91,7% der Theorie mit einer Reinheit (GC) von 96,1%; Fp. 75 bis 77°C.

### Beispiel 12

19,1 g (0,1 mol) 2,6-Diamino-3,4-dichlortoluol werden mit 500 ml Methanol, 15,4 g (0,2 mol) Ammoniumacetat und 2 g Platin auf Medizinalkohle (1%ig) in einem Autoklaven bei 150°C mit Wasserstoff bei einem Gesamtdruck von 180 bar hydriert. Nach Abtrennen des Kontaktes durch Filtrieren und Entfernen des Lösungsmittels wird der Rückstand in Wasser aufgenommen, mit Natronlauge neutral gestellt und mit Dichlormethan extrahiert. Man erhält 2,6-Diamino-4-chlortoluol in einer Ausbeute von 97,1% der Theorie, die Reinheit beträgt 95,3%.

### Beispiel 13

Es wird analog Beispiel 12 gearbeitet, statt des Ammoniumacetats werden 10,7 g (0,2 mol) Ammoniumchlorid zugegeben. Es wird partiell mit Wasserstoff bei einem Gesamtdruck von 20 bar und bei 90°C enthalogeniert. Man erhält 2,6-Diamino-4-chlortoluol in einer Reinheit von 98% und einer Ausbeute von 91% der Theorie.

### Beispiel 14

25,1 g (0,1 mol) 3,4-Dichlor-2,6-dinitrotoluol werden mit 200 ml Isopropanol und 2 g Platin auf Kohle (1%ig) versetzt und in einem mit Rührer versehenen Autoklaven bei 120°C mit Wasserstoff bei einem Gesamtdruck von 10 bar hydriert. Nach Verlangsamung der Wasserstoffaufnahme wird der Druck bei gleichen Temepraturbedingungen auf 50 bar erhöht und bei diesen Bedingungen gehalten, bis die partielle Entchlorierung vollständig abgelaufen ist. Die Aufarbeitung erfolgt analog zu Beispiel 12. Der Gehalt des Hydrierproduktes an 2,6-Diamino-4-chlortoluol beträgt 97,4%.

### Beispiel 15

19,1 g (0,1 mol) 2,6-Diamino-3,4-dichlortoluol, 10,7 g (0,2 mol) Ammoniumchlorid und 500 ml Wasser werden im Autoklaven mit 50 mg Platinoxid versetzt. Die Umsetzung erfolgt bei 90°C mit Wasserstoff bei einem Gesamtdruck von 20 bar. Nach Abtrennen des Kontaktes durch Filtrieren wird mit Natronlauge neutral gestellt und mit Dichlormethan extrahiert. Der Gehalt an 2,6-Diamino-4-chlortoluol im Hydrierprodukt beträgt 89%.

*Beispiel 16*

25,1 g (0,1 mol) 3,4-Dichlor-2,6-dinitrotoluol, 25,2 g (0,45 mol) Eisenpulver werden mit 50 ml Wasser vorgelegt und bis zum Kochpunkt erwärmt. Anschliessend werden 100 ml konzentrierter Salzsäure zugetropft und das Reaktionsgemisch unter Rückfluss gehalten bis das 3,4-Dichlor-2,6-dinitrotoluol vollständig zum 2,6-Diamino-3,4-dichlortoluol umgesetzt ist. Nach Zugabe von weiteren 16,8 g (0,3 mol) Eisen wird bei Rückflusstemperatur enthalogeniert.

Nach Neutralstellen und Abtrennen des Eisenschlammes wird mit Dichlormethan extrahiert. Das Reaktionsprodukt enthält 88,9% 2,6-Diamino-4-chlortoluol.

## Patentansprüche

1. Verfahren zur Herstellung von gegebenenfalls p-chlorsubstituiertem 2,6-Diaminotoluol, dadurch gekennzeichnet, dass man 3,4-Dichlortoluol in Gegenwart eines inerten, mit Wasser nicht mischbaren, organischen Lösungs- und/oder Verdünnungsmittels bei Temperaturen von −10 bis +100°C dinitriert und anschliessend, gegebenenfalls nach Zwischenisolierung der Dinitroverbindung, unter teilweiser oder vollständiger Abspaltung der Chloratome in Gegenwart eines inerten organischen Lösungs- und/oder Verdünnungsmittels und/oder Wasser zur Diaminoverbindung reduziert.

2. Verfahren nach dem Anspruch 1, dadurch gekennzeichnet, dass man als inerte organische Lösungs- und/oder Verdünnungsmittel bei der Nitrierung aliphatische und/oder cycloaliphatische Kohlenwasserstoffe mit 5 bis 20 Kohlenstoffatomen und/oder halogenierte aliphatische Kohlenwasserstoffe mit 1 bis 3 Kohlenstoffatomen einsetzt.

3. Verfahren nach den Ansprüchen 1 und 2, dadurch gekennzeichnet, dass man als Nitriermittel Gemische aus Schwefelsäure und Salpetersäure, Gemische aus Oleum und Salpetersäure sowie Nitroniumsalze einsetzt.

4. Verfahren nach den Ansprüchen 1 bis 3, dadurch gekennzeichnet, dass man die Nitriermittel in Mengen von 2 bis 4 mol pro mol 3,4-Dichlortoluol einsetzt.

5. Verfahren nach den Ansprüchen 1 bis 4, dadurch gekennzeichnet, dass man bei der Reduktion als inerte organische Lösungs- und/oder Verdünnungsmittel aliphatische, cycloaliphatische, aromatische und/oder araliphatische Kohlenwasserstoffe mit bis zu 20 Kohlenstoffatomen und/oder aliphatische, cycloaliphatische und/oder aromatische Ether mit bis zu 14 Kohlenstoffatomen und/oder Phenole, aliphatische und/oder cycloaliphatische Alkohole mit bis zu 12 Kohlenstoffatomen und/oder aliphatische Carbonsäuren, Carbonsäureester und/oder Carbonsäureamide mit bis zu 12 Kohlenstoffatomen einsetzt.

6. Verfahren nach den Ansprüchen 1 bis 5, dadurch gekennzeichnet, dass man die Reduktion mit Wasserstoff katalytisch in Gegenwart von gegebenenfalls auf Trägern aufgebrachten Raney-Katalysatoren oder Edelmetallen der 8. Gruppe des Periodensystems der Elemente oder mit unedlen Metallen in saurem Medium durchführt.

7. Verfahren nach den Ansprüchen 1 bis 6, dadurch gekennzeichnet, dass man zur vollständigen Abspaltung der Chloratome als Katalysator Raney-Nickel oder Palladium einsetzt.

8. Verfahren nach den Ansprüchen 1 bis 6, dadurch gekennzeichnet, dass man zur partiellen Enthalogenierung als Katalysator Platin einsetzt.

9. Verfahren nach den Ansprüchen 1 bis 8, dadurch gekennzeichnet, dass man die katalytische Reduktion bei Wasserstoffdrücken von 1 bis 300 bar und Temperaturen von 20 bis 250°C durchführt.

10. Verfahren nach den Ansprüchen 1 bis 9, dadurch gekennzeichnet, dass man zur vollständigen Abspaltung der Chloratome bei der Reduktion des dinitrierten 3,4-Dichlortoluols Metalloxide, Metallhydroxide, Metallcarbonate und/oder Salze organischer Säuren dem Reaktionsgemisch zusetzt.

## Revendications

1. Procédé de préparation du 2,6-diaminotoluène éventuellement chloré en position para, caractérisé en ce que l'on soumet le 3,4-dichlorotoluène à dinitration en présence d'un solvant et/ou diluant organique inerte non miscible à l'eau à des températures de −10 à +100°C puis en ce que, éventuellement après isolement intermédiaire du composé dinitré, on soumet ce dernier à réduction en composé diaminé avec séparation partielle ou complète des atomes de chlore en présence d'un solvant et/ou diluant organique inerte et/ou d'eau.

2. Procédé selon la revendication 1, caractérisé en ce que l'on utilise en tant que solvants et/ou diluants organiques inertes à la nitration des hydrocarbures aliphatiques et/ou cycloaliphatiques contenant 5 à 20 atomes de carbone et/ou des hydrocarbures aliphatiques halogénés contenant 1 à 3 atomes de carbone.

3. Procédé selon les revendications 1 et 2, caractérisé en ce que l'on utilise, en tant qu'agents nitrants, des mélanges d'acide sulfurique et d'acide nitrique, des mélanges d'oléum et d'acide nitrique ou des sels de nitronium.

4. Procédé selon les revendications 1 à 3, caractérisé en ce que l'on met en œuvre l'agent nitrant en quantité de 2 à 3 mol par mole du 3,4-dichlorotoluène.

5. Procédé selon les revendications 1 à 4, caractérisé en ce que l'on utilise, en tant que solvants et/ou diluants organiques inertes à la réduction, des hydrocarbures aliphatiques, cycloaliphatiques, aromatiques et/ou araliphatiques contenant jusqu'à 20 atomes de carbone et/ou des éthers aliphatiques, cycloaliphatiques et/ou aromatiques contenant jusqu'à 14 atomes de carbone et/ou des phénols, des alcools aliphatiques et/ou cycloaliphatiques contenant jusqu'à 12 atomes de carbone et/ou des acides carboxyliques, des esters d'acides carboxyliques et/ou des amides d'acides carboxyliques aliphatiques contenant jusqu'à 12 atomes de carbone.

6. Procédé selon les revendications 1 à 5, caractérisé en ce que l'on effectue la réduction par hydrogénation catalytique en présence de catalyseurs de Raney ou de métaux nobles du huitième groupe de la classification périodique des éléments, éventuellement appliqués sur support, ou à l'aide de métaux non nobles en milieu acide.

7. Procédé selon les revendications 1 à 6, caractérisé en ce que, pour la séparation complète des atomes de chlore, on utilise en tant que catalyseur le nickel de Raney ou le palladium.

8. Procédé selon les revendications 1 à 6, caractérisé en ce que, pour la déshalogénation partielle, on utilise le platine en tant que catalyseur.

9. Procédé selon les revendications 1 à 8, caractérisé en ce que la réduction catalytique est effectuée sous des pressions d'hydrogène de 1 à 300 bar et à des températures de 20 à 250°C.

10. Procédé selon les revendications 1 à 9, caractérisé en ce que, pour séparer complètement les atomes de chlore à la réduction du 3,4-dichlorotoluène dinitré, on ajoute au mélange de réaction des oxydes métalliques, des hydroxydes métalliques, des carbonates métalliques et/ou des sels d'acides organiques.

## Claims

1. Process for the preparation of optionally p-chlorine-substituted 2,6-diaminotoluene, characterised in that 3,4-dichlorotoluene is dinitrated in the presence of an inert, water-immiscible, organic solvent and/or diluent at temperatures of −10 to +100°C and is subsequently reduced, optionally after intermediate isolation of the dinitro compound, in the presence of an inert organic solvent and/or diluent and/or of water, with partial or complete splitting-off of the chlorine atoms, to give the diamino compound.

2. Process according to Claim 1, characterised in that aliphatic and/or cycloaliphatic hydrocarbons with 5 to 20 carbon atoms and/or halogenated aliphatic hydrocarbons with 1 to 3 atoms are emloyed as inert organic solvents and/or diluents for the nitration.

3. Process according to Claims 1 and 2, characterised in that mixtures of sulphuric acid and nitric acid, mixtures of oleum and nitric acid, or nitronium salts are employed as nitrating agents.

4. Process according to Claims 1 to 3, characterised in that the nitrating agents are employed in amounts of 2 to 4 mol per mol of 3,4-dichlorotoluene.

5. Process according to Claims 1 to 4, characterised in that aliphatic, cycloaliphatic, aromatic and/or araliphatic hydrocarbons with up to 20 carbon atoms and/or aliphatic, cycloaliphatic and/or aromatic ethers with up to 14 carbon atoms and/or phenols, aliphatic and/or cycloaliphatic alcohols with up to 12 carbon atoms and/or aliphatic carboxylic acids, carboxylic acid esters and/or carboxylic acid amides with up to 12 carbon atoms are employed as inert organic solvents and/or diluents in the reduction.

6. Process according to Claims 1 to 5, characterised in that the reduction is carried out with hydrogen, catalytically in the presence of optionally carrier-supported Raney catalysts or noble metals of group 8 of the periodic table of the elements, or with base metals in an acidic medium.

7. Process according to Claims 1 to 6, characterised in that for completely splitting-off the chlorine atoms Raney nickel or palladium is emloyed as the catalyst.

8. Process according to Claims 1 to 6, characterised in that for partial dehalogenation platinum is employed as the catalyst.

9. Process according to Claims 1 to 8, characterised in that the catalytic reduction is carried out under hydrogen pressures of 1 to 300 bar and at temperatures of 20 to 250°C.

10. Process according to Claims 1 to 9, characterised in that, in order to split off the chlorine atoms completely during the reduction of the dinitrated 3,4-dichlorotoluene, metal oxides, metal hydroxides, metal carbonates and/or salts of organic acids are added to the reaction mixture.